# EUROPÄISCHE PATENTSCHRIFT

(11) **EP 0 879 408 B1**
(45) Veröffentlichungstag und Bekanntmachung des Hinweises auf die Patenterteilung: **13.09.2000**
(21) Anmeldenummer: 97902280.3
(22) Anmeldetag: 31.01.1997
(51) Int. Cl.: G01N 1/28, H05H 3/04, G01N 15/14

(54) **VERFAHREN ZUM SORTIEREN UND ZUR GEWINNUNG VON BIOLOGISCHEN OBJEKTEN AUF EINEM PLANAREN TRÄGER DURCH FREIPRÄPARIEREN MITTELS EINES LASERSTRAHLES UND ANSCHLIESSENDES WEGKATAPULTIEREN DURCH EINEN LASER-SCHUSS**
METHOD FOR THE CONTACTLESS LASER-ASSISTED MICROINJECTION, SORTING AND PRODUCTION OF BIOLOGICAL OBJECTS GENERATED IN A PLANAR MANNER
PROCEDE DE MICRO-INJECTION SANS CONTACT, DE TRI ET DE PRODUCTION D'OBJETS BIOLOGIQUES OBTENUS DE MANIERE PLANAIRE, A L'AIDE DE FAISCEAUX LASER

(30) Priorität: 05.02.1996 DE 19603996; 23.04.1996 DE 19616216
(43) Veröffentlichungstag der Anmeldung: 25.11.1998
(73) Patentinhaber: P.A.L.M. GmbH Mikrolaser-Technologie, 82347 Bernried (DE)
(72) Erfinder: SCHÜTZE, Karin, D-82515 Wolfratshausen (DE); SCHÜTZE, Raimund, D-82515 Wolfratshausen (DE)
(74) Vertreter: Ruschke, Hans Edvard, Dipl.-Ing.
(86) Internationale Anmeldenummer: EP9700429
(87) Internationale Veröffentlichungsnummer: WO9729355

(56) Entgegenhaltungen:
- WO-A-95/17656
- WO-A-95/23960
- DE-A- 4 300 698
- JOURNAL OF MICROSCOPY, Bd. 107, Mai 1976, Seiten 19-24, XP000671562 ISENBERG G ET AL: "Cell surgery by laser micro-dissection: a preparative method"
- PATENT ABSTRACTS OF JAPAN vol. 017, no. 394 (C-1088), 23.Juli 1993 & JP 05 076342 A (AGENCY OF IND SCIENCE & TECHNOL), 30.März 1993,
- BIOIMAGING, Bd. 1, 1993, GB, Seiten 1-5, XP000671315 SUGIURA T ET AL: "Photon -pressure exertion on thin film and small particles in the evanescent field"
- NATURE, Bd. 368, 14.April 1994, LONDON GB, Seiten 667-669, XP000647487 SCHÜTZE KARIN ET AL: "Catch and move - cut or fuse" in der Anmeldung erwähnt

## Beschreibung

Die Erfindung betrifft ein Verfahren zur Mikroinjektion sowie zum Sortieren und zur Gewinnung einzelner biologischer Objekte. Die Objekte sind hierbei auf einem festen planaren Träger nebeneinander angeordnet. Dieses Verfahren eignet sich dazu, bestimmte Substanzen in einzelne biologische Objekte z.B. Zellen zu mikroinjizieren und anschließend diese auszusortieren. Weiterhin können mit diesem Verfahren aus einer sehr großen Zahl von Objekten (z.B. 10⁵ - 10⁹) einzelne Objekte räumlich abgetrennt und ausgesondert werden. Die Abtrennung von gehäuften Zellen als Gesamteinheit ist ebenso möglich. Ebenso kann das Verfahren zur Separation von spezifischen Zellen aus Gewebeschnitten eingesetzt werden.

Voraussetzung für dieses Sortierverfahren ist die vorherige Erkennung und Selektion der betreffenden Objekte aufgrund spezifischer Eigenschaften (z.B. durch Färbung, Fluoreszenzmarkierung oder durch radioaktive Markierung). Unter "biologischen Objekten" werden im Rahmen der vorliegenden Anmeldung vor allem lebende oder fixierte biologische Zellen oder Zellbestandteile verstanden.

Zur Materialinjektion in lebende Zellen wurden üblicherweise Mikrokapillaren verwendet, die über einen meist pneumatisch oder hydraulisch bewegten Mikromanipulator gesteuert werden. Die gewünschten Substanzen werden unter großer mechanischer Belastung in die einzelne Zelle injiziert. Die Herstellung der sterilen Mikrokapillaren ist zeitaufwendig und kostenintensiv.

Tsukakoshi et al. (1984) sowie Tao et al. (1987) verwendeten einen fokussierten Laserstrahl, um kleine selbstheilende Löcher ohne mechanischen Kontakt in die Zellmembran zu bohren. Die kurze Öffnungszeit reicht aus, um das in der umgebenden Flüssigkeit gelöste Material in die Zelle einzuschleusen. Die größte Effizienz bei der Lasermikroinjektion von genetischem Material wurde erzielt, wenn der Laser das Loch direkt in den Zellkern schießt.

Problem bei dieser Methode ist, daß für eine präzise Lasermikroinjektion im Submicronbereich die Zielobjekte sowohl lateral, also in x- und y-Richtung, als auch vertikal, also in z-Richtung, mit Nanometer-Genauigkeit angefahren werden müssen. Für ein automatisiertes Mikroinjizieren müssen zudem die relevanten Zielzellen über ein bildanalytisches Verfahren erkannt, exakt in der Laserschußlinie positioniert und vor allem in z-Richtung exakt fokussiert werden.

Ein weiteres Problem besteht darin, die erfolgreich injizierten Zellen von den anderen Zellen zu isolieren bzw. für die weiteren Untersuchungen zu präparieren.

Zur Separation einzelner biologischer Objekte lassen sich Objekte mit optischen Methoden, wie der optischen Pinzette (Optical Tweezer) in einer wässrigen Lösung bewegen (K. Schütze, A. Clement-Sengewald, Nature, 667 (Vol. 368) 1994). Aufgrund der geringen Kraftübertragung ist diese Methode auf Objekte beschränkt, die sich frei in der Lösung bewegen können. Da sich die sortierten wie die unsortierten Objekte in der gleichen Lösung befinden, ist eine getrennte Kultivierung nur mit zusätzlichem Aufwand erzielbar. Für eine getrennte Kultivierung müssen diese Zellen mit einer anderen Methode wie z.B. mit Mikrokapillaren abgetrennt bzw. abgesaugt werden. Adhärent wachsende Zellen oder fixierte Zellen auf einem Schnittpräparat können mit feinen Nadeln, die über Mikromanipulatoren bewegt werden, separiert werden. Hierbei werden die Zellen direkt berührt und könnten somit mechanisch belastet werden. Zudem besteht die Gefahr der Kontamination durch ungewolltes Zellmaterial. Beide Methoden sind verhältnismäßig zeitintensiv, so daß sie nicht zur Bearbeitung einer Vielzahl von Objekten geeignet sind.

Zur Separierung einzelner Zellen aus einer großen Zahl (> 10⁶) in einer Flüssigkeit dispergierter, biologischer Objekte geeignete Trenn- bzw. Sortierapparate sind kommerziell erhältlich. Während bei der fluoreszenzaktivierten Zellsortierung (FACS = **F**luorescence **a**ctivated **C**ell **S**orter) elektrostatische Prinzipien zur räumlichen Separation zum Einsatz kommen, arbeitet der magnetisch aktivierte Zellsortierer (MACS = **M**agnetic **a**ctivated **C**ell **S**orter) mit magnetischen Kräften. Hierbei liegen die Zellen jedoch nicht auf einem planaren Träger nebeneinander. Überdies haben beide Methoden den Nachteil, daß sich manche Objekte nur eingeschränkt (FACS) oder überhaupt nicht getrennt voneinander absondern lassen (MACS).

Die vorgestellten Methoden können keine einzelnen Zellen aus einem Zellverband wie etwa einem Gewebe oder aus histologischen Gewebepräparaten lösen.

Aus der JP-A-05 076342 ist eine Vorrichtung zum Fangen und Sammeln von mikroskopischen Objekten wie etwa Zellen bekannt. Dabei werden die interessierenden Objekte von zwei einander gegenüberliegenden kontinuierlichen Fanglasern eingefangen und an einem Ort gehalten, woraufhin sie von einem dritten kontinuierlichen Laser entlang seines Strahls zu einer Auffangvorrichtung geführt werden. Aus dem Artikel " Cell surgery by laser micro-dissection: a preparative method" im *Journal of Microscopy,* Vol. 107 (1976) ist eine Vorrichtung bekannt, die einen quasi-kontinuierlichen N₂-Laser zur Mikrodissektion verwendet, sowie ein Mikroskop zum anschliessenden Betrachten der ausgewählten Objekte.

Ferner sind unter dem Namen "Ablative Photodecomposition" Verfahren bekannt, bei denen mit gepulsten UV-Lasern, insbesondere mit Excimer-Lasern, ein gezielter Materialabtrag bei Polymeren erfolgt. Diese Verfahren können im weitesten Sinne als Ätzverfahren angesehen werden. Ein ähnliches Verfahren, bei dem jedoch ein kontinuierlich betriebener UV-Laser verwendet wird, wird in dem US-Patent 5 211 805 beschrieben. Dieses Verfahren soll sich zur industriellen Bearbeitung von technischen Polymeren und zur biomedizimischen Behandlung von biologischem Gewebe eignen. Hiermit ist ein Sortieprinzip verwandt, das mit Laserstrahlen die auf einem Träger befindlichen unerwünschten biologischen Objekte mit hohen Strahlungsdosen zerstört, während die selektierten (erwünschten) Objekte zurück bleiben (US 4 624 915). Dieser Prozeß ist verhältnismäßig aufwendig, um einzelne Objekte aus großen Populationen zu selektieren.

Die der Erfindung zugrundeliegende Aufgabe besteht u.a. darin, ganz gezielt bestimmte biologische Objekte mit einer ausgewählten Substanz durch berührungslose Laser-Mikroinjection zu beladen und anschließend die erfolgreich injizierten Objekte auszusortieren. Die biologischen Objekte können nebeneinander auf einem festen planaren Träger wie z. B. einer polymeren Trägerfolie ausgebracht sein. Hierbei soll der Vorgang des Absonderns möglichst kurz (< 10 s) und berührungslos, z.B. in abgetrennten, sterilen Kammern durchführbar, sein. Außerdem soll der Prozeß sehr zuverlässig und damit in einfacher Weise automatisierbar sein. Gleichzeitig soll die Überlebensfähigkeit bzw. die Morphologie der biologischen Objekte in der Regel gewährleistet bleiben; d.h. die biologischen Objekte sollen durch den Mikroinjektionsvorgang und durch den Abtrennprozeß nicht geschädigt bzw. beeinträchtigt werden.

Die Aufgabe der Mikroinjektion wird erfindungsgemäß dadurch automatisiert durchgeführt, daß ein Objektfeld auf dem Deckglas oder einer Trägerfolie mit der motorisierten, computergesteuerten Mikroskopbühne durch mäanderförmiges Abrastern abgefahren wird. Dabei wird die einzelne Zielzelle (Zielobjekt) durch ein bildanalytisches Verfahren über Farb- oder Mustererkennung ausgewählt und mittels x/y-Verschiebung in den Bereich des Laserschusses gebracht. Anschließend wird auf die Zelle bzw. die gewünschte Zellstruktur (in z-Richtung) fokussiert und mit einem gezielten Laserschuß die Zelle mikroperforiert. Die Bewegung der Objekte in x/y-Richtung kann entweder über die Achsen des Mikroskoptisches, die z-Verschiebung durch eine dritte Achse des Mikroskoptisches oder aber durch die Fokusverstellung des Objektives am Mikroskop selbst per Computersteuerung eingestellt werden. Alternativ kann eine Einstellung auf das Zielobjekt auch bei fixiertem Mikroskoptisch durch eine dreidimensionale, computergesteuerte Laserfokusbewegung erfolgen. Um die erfolgreich mikroinjizierten Zellen anschließend aus dem übrigen Zellrasen auszusortieren, wird das nachfolgend beschriebene Selektionsverfahren eingesetzt.

Die Aufgabe der Selektion und Abtrennung wird erfindungsgemäß dadurch gelöst, daß ein Objektfeld der Trägerfolie, auf dem sich das selektierte biologische Objekt bzw. der histologische Schnitt befindet, mit einem Laserstrahl ausgeschnitten wird und durch einen laserinduzierten Transportprozeß auf ein unmittelbar oberhalb oder unterhalb der Trägerfolie angeordnetes Auffängersubstrat übertragen wird. Die erfindungsgemäße Lösung besteht also darin, daß die biologischen Objekte zuerst entweder visuell mit dem Auge ausgesucht oder aber über die Farb- bzw. Mustererkennung eines bildanalytischen Verfahrens aufgesucht und anschließend in einem dem Präparat angepaßten, z.B. auch kreisförmigen, Umfeld mitsamt dem Träger von einem Laserstrahl ausgeschnitten werden und anschließend aus der Trägerfolie heraus auf einen in der Nähe angebrachten Auffänger geschleudert werden. Es wurde beobachtet, daß die herausgetrennten Objektfelder dabei stets in Richtung des Laserstrahls beschleunigt werden. Eine physikalische Erklärung für diesen laserinduzierten Transportprozeß liegt möglicherweise in dem photokinetischen Impuls, der von dem Laserstrahl auf das ausgeschnittene Objektfeld übertragen wird und damit für die Beschleunigung verantwortlich ist. Die räumliche Separation der biologischen Objekte beruht also bei diesem Prozeß auf dem Ausschneiden der gewünschten Objektfelder mit den vorher selektierten Objekten und ihrem Abtransport zu einem in der Nähe befindlichen Auffängersubstrat.

Das Ausschneiden des Objektfeldes kann vorteilhaft in der Weise erfolgen, daß der Laserstrahl durch eine Relativbewegung von Laserstrahl und Trägerfolie auf einer geschlossenen, das Objektfeld einschließenden Kurve um das biologische Objekt herumgeführt wird. Alternativ kann aber die Abtrennung eines Objektfeldes in Analogie zu einem Stanzprozeß auch so durchgeführt werden, daß der das Objektfeld einschließende Schnittbereich durch eine mit dem Laserstrahl beleuchtete, auf die Trägerfolie abgebildete Schlitzmaske simultan belichtet wird.

Wie schon erwähnt, soll das Auffängersubstrat in unmittelbarer Nähe der Trägerfolie angeordnet werden, so daß die Transportwege bei dem Separationsprozeß kurz sind. Gute Ergebnisse wurden erzielt, wenn die Entfernung 0,5 bis 10 mm, vorzugsweise 1 bis 3 mm betrug.

Der Durchmesser des Objektfeldes mit dem selektierten Objekt kann aufgrund des außerordentlich präzisen Schnittvorgangs sehr klein, d.h. in einem Bereich von 10 µm bis 20 um, gewählt werden.

Zum Ausschneiden wird vorzugsweise ein UV-Laser verwendet, wobei der Laserstrahl fokussiert auf die Trägerfolie abgebildet wird.

Die Trägerfolie besteht dabei aus einer UV-absorbierenden Polymerfolie mit einer Dicke zwischen 5 µm und 15 µm, deren Absorptionsverhalten an die Wellenlänge des UV-Lasers angepaßt ist, also zumindest in der Umgebung der Laserwellenlänge ein Absorptionsmaximum besitzt. Als besonders geeignet haben sich Polymerfolien erwiesen, die mindestens 5 Gew.-% eines aromatischen oder teilaromatischen Polykondensats enthalten. Die geometrische Form des Auffängersubstrats ist relativ unkritisch. Geeignet ist z.B. eine relativ dicke Folie oder Platte, die im Abstand von 0,5 bis 10 mm oberhalb oder unterhalb von der Trägerfolie parallel dazu angebracht wird. Das Auffängersubstrat kann aber auch als topfförmiger Behälter ausgebildet sein. Insbesondere werden Mikrozentrifugenbehälter empfohlen, wie sie in der Molekularbiologie verwendet werden, z.B. eine Mikrotiterplatte mit 90 bis 500 Vertiefungen (Wells).

Gemäß einer speziellen Ausführungsform ist die Platte oder Folie mit einer adhäsiven Schicht versehen. Durch eine solche Klebeschicht können die abgeschleuderten Objektfelder auf dem Auffängersubstrat fixiert werden.

Zur Erkennung und Selektion der gewünschten biologischen Objekte auf der Trägerfolie kann vorzugsweise die Methode der Fluoreszenzspektroskopie eingesetzt werden Alternativ können die biologischen Objekte mit Hilfe bekannter histochemischer Farbreaktionen oder morphologisch wahrnehmbarer Veränderungen entweder visuell oder aber über bildanalytisches Verfahren per Computer (digital?) erkannt und anschließend selektiert werden.

Gemäß einer Weiterentwicklung werden die biologischen Objekte mit einem flüssigen, für die Laserstrahlung durchsichtigen Nähr- oder Puffermedium beschichtet. Unter dieser Bedingung können selektierte Objektfelder erfindungsgemäß ausgeschnitten und abgelöst werden.

Das erfindungsgemäße Separierverfahren wird vorteilhaft in einem abgeschlossenen System durchgeführt. Zu diesem Zweck wird sowohl die Trägerfolie mit den zu sortierenden Objekten, als auch das Auffängersubstrat in einem geschlossenen Behälter untergebracht, der ein UV-transparentes Fenster für den Laserstrahl besitzt.

Im folgenden wird die Erfindung anhand von Zeichnungen und Ausführungsbeispielen näher erläutert.

Es zeigen:
- Fig. 1: schematisch eine Trägerfolie mit adherierten Bakterien,
- Fig. 2: den prinzipiellen Aufbau einer Apparatur zur Realisierung des erfindungsgemäßen Verfahrens,
- Fig. 3/4: das zugrundeliegende Sortierprinzip,
- Fig. 5/6: den Aufbau mit aufrechtem/inversem Mikroskop und
- Fig. 7: das Ablegen des Obektes in einem Auffanggefäß.

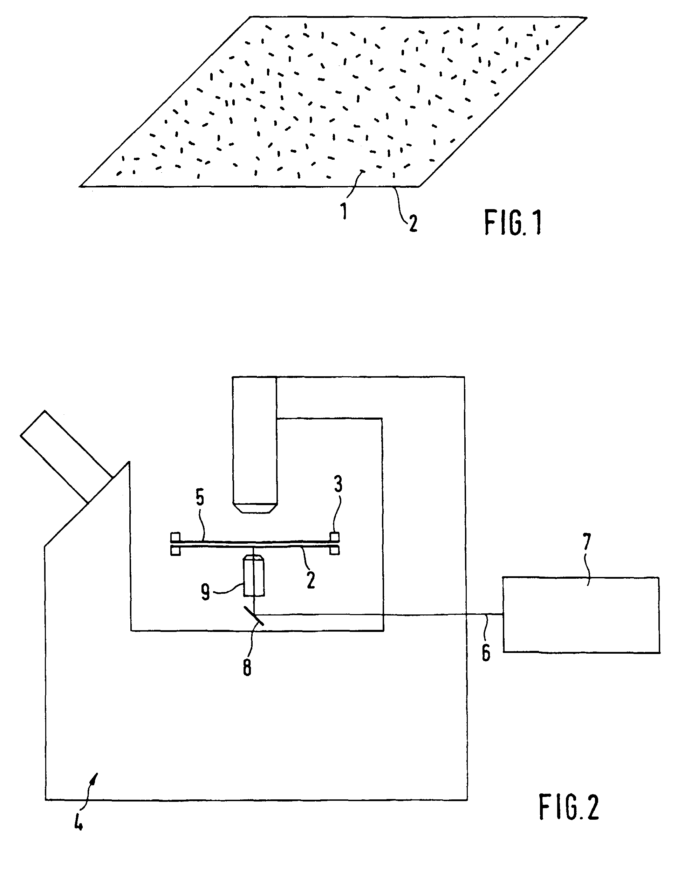

Fig. 1 zeigt beispielsweise eine Bakterienpopulation 1, die planar auf einer 5 µm starken Polyarylatfolie 2 (Trägerfolie) ausgebracht ist. Zum Sortiervorgang wird die Trägerfolie 2 in einen Schiebetisch 3 eingelegt und mechanisch fixiert. Dieser Tisch befindet sich gem.- Fig. 2 als Objekttisch in einem inversen Mikroskop 4 und kannz.B. mittels eines computergesteuerten Schrittmotors in x,y-Richtung (Horizontalebene) positioniert werden. Im Schiebetisch 3 ist ferner gegenüber der Trägerfolie 2 im Abstand von 1,8 mm ein plattenförmiges Auffängersubstrat 5 gehaltert. Bei einer Bewegung des Schiebetisches 3 werden also gleichzeitig die Trägerfolie 2 und das Auffängersubstrat 5 senkrecht zum Strahlengang (z-Richtung) im Mikroskop verschoben. Der Schiebetisch 3 mit der Trägerfolie 2 und den darauf befindlichen biologischen Objekten 1, sowie das Auffängersubstrat 5 sind von einem geschlossenen Gehäuse mit einem UV-durchlässigen Fenster für den Laserstrahl umgeben (nicht dargestellt), so daß das Verfahren in einem hermetisch abgeschlossenen System durchgeführt werden kann.

Als Trägerfolie für die biologischen Objekte wird eine 5 µm bis 15 µm dicke, UV-absorbierende Polymerfolie aus einem Polymer verwendet, das mindestens 5 Gew.-% eines aromatischen oder teilaromatischen Polykondensates, wie z.B. Polycarbonate, Polyurethane, Polyarylate, Copolyester, Polyestercarbonate oder Blends aus diesen Polykondensaten und anderen Thermoplasten enthält. Andersartige Folien sind in diesem Zusammenhang denkbar.

Zur räumlichen Trennung z.B. einer einzelnen Bakterie aus der ausgebrachten Population wird ein UV-Laserstrahl 6 der Wellenlänge 337 nm eines gepulsten N₂-Lasers 7 verwendet. Der Laser 7 liefert bei einer maximalen Pulsfrequenz von 20 Hz ca. 300 µJ Strahlenergie. Geeignet sind auch andere gepulste oder kontinuierlich arbeitende Laser wie z.B. Excimerlaser mit einer Wellenlänge von 193, 248 oder 308 mm oder ein frequenzvervierfachter Nd:YAG-Laser mit einer Wellenlänge von 266 nm oder ein frequenzverdoppelter Ar-Ionenlaser mit Wellenlängen von 244 nm oder 257 nm. Der Laserstrahl 6 wird über einen dielektrischen Strahlteiler 8 und ein verkleinerndes Mikroskopobjekt 9 (Verkleinerung 63 x, Öffnungsverhältnis NA = 0.9, oder auch andere Objektive) auf die Trägerfolie 2 punktförmig abgebildet. Dieser Punkt hat einen Durchmesser von mindestens 1 µm.

Eine kreisförmige bzw.geschlossene Schnittlinie mit einem Durchmesser von z.B. 10 µm wird um das selektierte Bakterium durch eine entsprechende Bewegung des Schiebetisches 3 in der Horizontalebene erzeugt. Die durch die Schnittlinie definierte Fläche stellt in diesem Fall die Objektfläche dar. Der Laserstrahl bleibt während des nachfolgend beschriebenen Schneidprozesses ortsfest.

Im Experiment betrug die relative Bahngeschwindigkeit des Laserstrahls zum Ausschneiden einer geschlossenen Fläche 5µm/s. Dabei entstand eine scharfkantige, eng begrenzte Schnittlinie, wobei gleichzeitig mit der Rückkehr des Laserstrahls zum Startpunkt der Schnittlinie ein Abriß der ausgeschnittenen Fläche erfolgte. Die Fläche wurde nun durch einen laserinduzierten Transportprozeß, dessen physikalische Wirkungsweise im Einzelnen noch nicht geklärt ist, von der Trägerfolie 2 weg auf das darüber befindliche, mit einer adhäsiven Klebeschicht versehene Auffängersubstrat 5 geschleudert und blieb dort haften. Eine andere Möglichkeit besteht darin, als Auffängersubstrat eine handelsübliche Mikrotiterplatle mit z.B. 96 Wells zu verwenden. Unter "Wells" werden in der pharmazeutischen Forschung die in der Mikrotiterplatte befindlichen Aussparungen bzw. kreisrunden Vertiefungen zur Probenaufnahme mit einem Durchmesser von ca. 4 mm und einer Tiefe von ca. 6 mm verstanden.

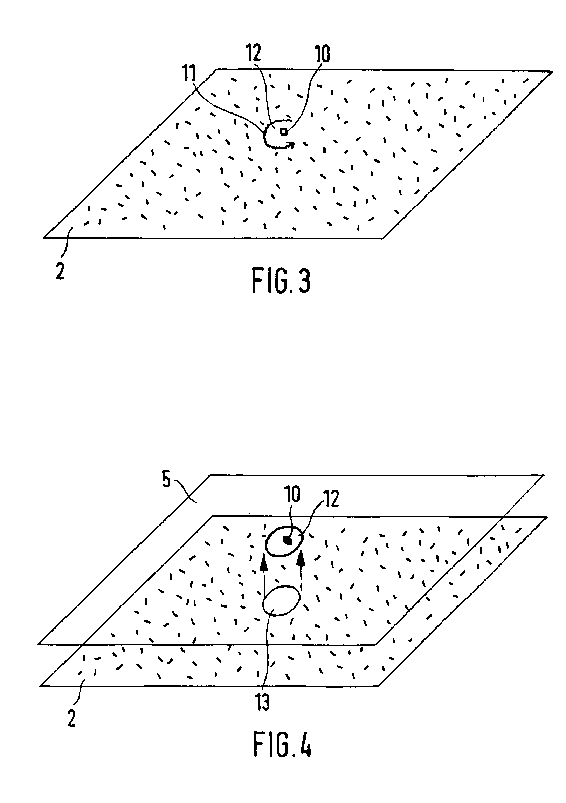

Der Sortierprozeß soll noch einmal anhand der Figuren 3 und 4 veranschaulicht werden. Figur 3 zeigt ein räumlich zu separierendes Bakterium 10 auf der Trägerfolie 2. Auf Grund der kreisförmigen Bewegung des Schiebetisches 3 ist durch den ortsfesten Laserstrahl 6 beim Schneidprozeß bereits eine Schnittlinie 11 von etwa 5 - 7 µm Breite in die Folie 2 geschrieben worden. Die Schnittbreite ist bedingt durch das Absorptionsverhalten der Folie. In diesem Bereich ist das Folienmaterial komplett abgetragen worden. Unmittelbar nachdem die Schnittlinie 11 zu einem geschlossenen Kreis vervollständigt worden ist, wird das abgetrennte Folienstück (Objektfeld) 12 mit dem darauf befindlichen Bakterium 10 in Richtung des Laserstrahls beschleunigt und wie in Fig. 4 dargestellt, auf das Klebeband 5 (Auffängersubstrat) geschleudert. In der Trägerfolie 2 verbleibt ein kreisrundes Loch 13.

Anstelle des Schiebetisches kann auch mit einem ruhenden Objektisch gearbeitet und der Laserstrahl mit Hilfe eines in den Laserstrahl eingebrachten geeigneten optischen Ablenkelementes auf einem Kreis um das selektierte Bakterium herumgeführt werden.

Voraussetzung für die Laser-Separation ist die vorherige Erkennung und Selektion der aus der Trägerfolie zu entfernenden Objektfelder. Eine in der pharmakologischen Forschung häufig angewandte Methode der Erkennung und Selektion von bestimmten Zellstrukturen ist die Fluoreszenzspektroskopie. Zu diesem Zweck wird ein kommerziell erhältliches Fluoreszenz-Mikroskop eingesetzt. Grundlage ist dabei, daß die für die Selektion vorgesehenen Zellen bzw. Bakterien ein signifikantes Fluoreszenzsignal erzeugen, das als Unterscheidungskriterium herangezogen wird. Mit Hilfe eines mit einem Suchalgorithmus ausgestatteten Scanprogramms kann dann der Schiebetisch 3 so gesteuert werden, daß automatisch nacheinander die Bereiche mit selektierten Bakterien als Objektfelder zentral im Gesichtsfeld des Fluoreszenzmikroskops positioniert und anschließend ausgeschnitten werden.

Zur Erkennung von biologischen Objekten in Gewebeschnitten können auch die bekannten histologischen Farbreaktionen oder im Mikroskop wahrnehmbare morphologische Veränderungen herangezogen werden.

Die Trägerfolie 2 kann auch mit einer für die Laserstrahlung durchlässigen Nähr- oder Pufferlösung, z.B. einer PBS-Pufferlösung, beschichtet werden.

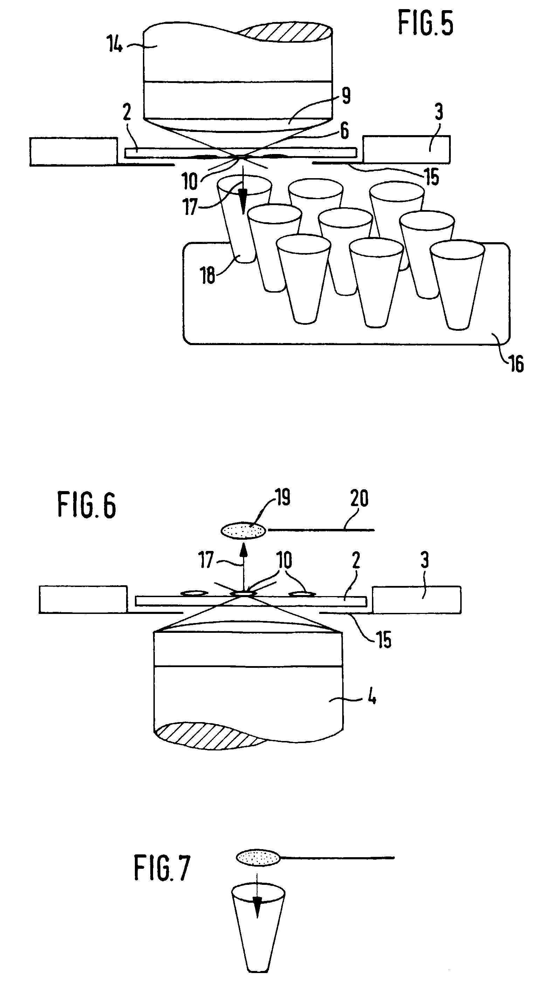

Gemäß einer Weiterentwicklung des Verfahrens kann die Lösung der Aufgabe dadurch erreicht werden, daß der Lichtdruck des Laserstrahls nun dafür verwendet werden soll, das gewünschte Partikel bzw. biologische Objekt selbst unmittelbar von der Oberfläche des festen planaren Trägers (Objektträger oder Petrischale) wegzukatapultieren und in einem geeigneten Gefäß aufzufangen, d.h. die Separierung eines biologischen Objektes ist also auch mit oder ohne gleichzeitiges Herauslösen bzw. Herausschneiden des das biologische Objekt tragenden Bereiches der Trägerfolie möglich. Dies geschieht nach der Weiterentwicklung in der folgenden Weise, die anhand der beiliegenden Figuren 5 bis 7 erläutert werden soll.

Bei Verwendung eines aufrechten Mikroskopes 14 gemäß Figur 5 wird ein geeigneter Objektträger 2 (Dicke ca. 170 µm) umgekehrt auf die Aufnahmehalterung 15 eines speziell für die Lasermikromanipulation entwickelten Mikroskoptisches 3 gelegt, d.h. das biologische Objekt 10 befindet sich auf der Unterseite des Objektträgers 2. Bei dem Objekt handelt es sich z.B. um histologische Gewebeschnitte von wenigen µm Dicke, oder um adherierende Chromosomen- bzw. DNA-Präparate.

Der Mikroskoptisch 3 ist für eine Verschiebung in 2 (für x/y Bewegung) oder 3 Achsen (für x/y/z Bewegung) ausgelegt und mit einer Aufnahmevorrichtung 15 für z.B. Objektträger 2 oder Petrischälchen versehen. Der Tisch 3 ist also über geeignete Antriebe motorisiert, die computergesteuert in bekannter Weise, z.B. über eine Computermaus (oder Joystick), bewegt werden können. Die hybriden Schrittmotoren der Achsen-Antriebe arbeiten mit einer hohen Präzision. Der kleinste Schritt beträgt 20 nm, der maximale Verfahrweg des Mikroskoptisches 3 kann bis zu mehreren Zentimetern (z.B. 10 cm) betragen. Die Präzision zum Wiederauffinden gespeicherter Punkte ist <400 nm. Es können Geschwindigkeiten zum Verfahren des Mikroskoptisches von wenigen um bis zu mehreren mm pro Sekunde gewählt werden. Mit einem sog. Framegrabber wird das über eine Videokamera aufgezeichnete, aktuelle Mikroskopbild auf einen Monitor gegeben und kann mit Computerfunktionen, z.B. Befehlsfunktionen, Steuerzeichen, Kontrollmarken etc., graphisch überlagert werden (Video-Overlay).

Für Lasermikromanipulation geeignet sind nur dünne Objektträger (ca. 170 µm dick) oder Petrischalen mit dünner (ca. 25 µm), gasdurchlässiger Folie (sog. Petriperm-Schälchen). Da für die Lasermikromanipulation im Nanometer-Bereich hohe Anforderungen an eine präzise Probenhalterung und Probenführung gestellt werden, wurde die Aufnahmehalterung 15 speziell konfiguriert: Bei den dünnen Objektträgern 2 besteht die Gefahr, daß sie sich z.B. bei Verwendung von Öl-Immersions-Objektiven leicht durchbiegen und somit keine gute Fokussierung erreicht werden kann. Um dies zu vermeiden, muß der Objektträger 2 an mindestens 3 Seiten der Halterungen aufliegen, wobei nur die beiden schmalen Seiten des Objektträgers 2 mit je einer Federklammer festgeklemmt werden können. Eine weitere Notwendigkeit, speziell für die Lasermikroskopie, ist die exakte Justierbarbeit des Probenhalters (Objektträger 2 oder Aufnahmehalterung 15). Es muß gewährleistet sein, daß die Probe immer im gleichen Abstand zur Spitze des Objektivs 9 liegt, und zwar im gesamten Verfahrbereich des Trägers (circa 5 - 10 cm).

Zuerst werden geeignete biologische Objekte 10 mit einer kleineren Vergrößerung lichtoptisch ausgewählt. Sobald alle interessanten Objekte im Computer gespeichert sind, wechselt man zu einem höhervergrößernden Objektiv. Den durch den Objektivwechsel bedingten Strahlversatz (chromatische Abberation) gleicht man durch eine Korrekturfunktion an einem gespeicherten Wert aus, die dann automatisch auf alle gespeicherten Punkte übertragen wird.

Der Computer verfährt nun zu dem ersten Objekt 10. Das von einer Videokamera aufgezeichnete Mikroskopbild wird auf dem in den Figuren nicht gezeigten Computermonitor dargestellt. Ein Marker auf dem Monitor zeigt die Position des Laserstrahlfokusses an. Die Mikroskopbühne wird entweder per Hand (über Maus oder Joystick kontrolliert) bewegt oder fährt automatisch durch ein Computerprogramm gesteuert nach vorgegebenem Muster im wesentlichen kreis- oder spiralförmig um das gewählte Objekt 10 herum. Der "Marker" auf dem Monitor kann als "Stift" betrachtet werden, mit dem die Kontur des gewünschten biologischen Objektes nachgezeichnet wird. Wenn gleichzeitig der Laser mit einer Pulsfrequenz von circa 15 - 20 Hz feuert, wird alles Material, das sich in der Schußlinie im Bereich des "Marker" befindet, abgetragen bzw. zerstört. Der extrem fokussierte Laserstrahl "zeichnet" eine feine Linie von circa 500 nm Breite rund um das gewünschte Objekt 10 und trennt es somit von seiner Umgebung. Bei Zellen im histologischen Schnitt löst sich durch diese Prozedur die gewünschte Zelle vom Zellverband und lockert sich vom Untergrund in Gestalt des durch die Schnittlinie gegebenen Objektfeldes. Durch das oben erwähnte spiralförmige Umrunden der ausgewählten Zielzelle kann der freigelaserte Bereich rund um die Zelle vergrößert werden.

Bei dem hier verwendeten Laser handelt es sich z.B. um einen gepulsten, kompakten Stickstofflaser von hoher Strahlqualität (Wellenlänge: 337 nm, Pulsdauer: 3 nsec, Pulsfrequenz: von 10 bis 30 Hertz). Andere Laser sind auch denkbar, sofern die verwendete Laserwellenlänge das biologische Material nicht negativ beeinflußt. Der Laserstrahl selbst bzw. seine Quelle bleibt vorzugsweise unbeweglich. Allerdings kann der Laser auch in x/y Richtung mit einem Radius von mehreren um relativ zur Objektebene bewegt werden, d.h. letztendlich ist nur wichtig, daß Laserstrahl und Objektebene (Mikroskop tisch) relativ zueinander bewegt werden.

Das auf diese Weise freipräparierte Objekt kann nun schneller und sicherer als im Stand der Technik (etwa mit einer Nadel) und vor allem berührungslos, d.h. wenn nötig auch völlig steril, mit einem weiteren, gezielten Laserschuß automatisch in ein Probengefäß hinein katapultiert werden (Flugbahn 17).

Dazu ist es notwendig, daß eine zweite Aufnahmehalterung 16 (z.B. um ein Auffanggefäß 18 oder eine Mikrotiterplatte einzuspannen) über zwei motorisierte und computergesteuerte Achsen so unter die erste Aufnahmehalterung 15 verfahren wird, daß das per Laser freipräparierte Objekt 10 genau über dem Auffanggefäß 18 plaziert wird. Hier ist ebenfalls eine hohe Präzision der Motorbewegung Voraussetzung für ein sauberes Aufsammeln der gewünschten Objekte. Ein einzelner, gezielter Laserschuß (eventuell leicht defokussiert) schleudert das selektierte biologische Objekt 10 bzw. die Zelle in Strahlrichtung (Flugbahn 17) ins Auffanggefäß 18. Danach kann ein neues Objekt ausgeschnitten werden und der ganze Vorgang wiederholt sich.

Zur Beschleunigung des Sammelvorgangs können alle gewünschten Objekte zuerst mit dem Laser freipräpariert werden. Danach wird das Auffanggefäß 18 unter den Mikroskoptisch gefahren. Die Mikroskopbühne fährt nacheinander die gespeicherten, lasermikrodissektierten Objekte wieder an. Je ein Schuß befördert die einzelnen Objekte nacheinander in jeweils frische (neue) Auffanggefäße 18, Fig. 5, die koordiniert mit der Bewegung der Mikroskopbühne 3 weitertransportiert werden. Es können aber auch mehrere Objekte in einem Gefäß gesammelt werden.

Bei einem inversen Mikroskop (Fig. 6) kann ein klebendes Scheibchen (Klebefolie, Agar bestrichener Träger etc.), das wenige µm direkt über das ausgeschnittene Objekt geführt wird, zum Auffangen des wegkatapultierten Objektes verwendet werden. Dies kann z.B. eine Klebeklatsche 19 sein, die dann vom Robotarm 20 in ein geeignetes Gefäß (Fig. 7) geworfen wird, und der Robotarm 20 sucht sich ein neues Klebeteilchen (siehe Fig. 6).

Der Vorleil des Laser-induzierten Separationsprozesses von Zellen besteht in der gezielten und gleichzeitig schnellen Manipulation von einzelnen Zellen im Vergleich zum Stand der Technik. Aufgrund des einfachen Prinzips ist der Prozeß sehr robust und einfach zu handhaben und eignet sich daher zur computergestützen automatischen Separation einer Vielzahl von biologischen Objekten. Ein unter sicherheitstechnischen Aspekten wichtiger Vorteil liegt ferner darin, daß der Separationsprozeß in einem hermetisch abgeschlossenen System durchgeführt werden kann, so daß die Umgebung vor pathogenen Zellen geschützt werden kann. Außerdem werden die Zellen vor Verunreinigungen aus der Umgebung geschützt.

Das Verfahren eignet sich prinzipiell zum Einsatz in Teilgebieten der Biotechnologie, Molekularbiologie und Pathologie, wo spezifische Zelltypen angereichert werden sollen. Beispielsweise können mit dem Green fluorescent Protein (GFP) als Reportergen transfizierte Zellen identifiziert werden. Marshall et al. (Neuron, Vol. 14, 211-215 (1995) benutzt z.B. die GFP-Methode, um die Expression von Ionenkanälen abzuschätzen. Gemäß einer weiteren Anwendung können für neurologische Experimente aus Gehirngewebeschnitten z.B. Gliazellen separiert werden. Hierzu werden fluoreszenzmarkierte Antikörper benutzt, um die Zellen zu identifizieren. Zur Diagnose könnten aus Gewebeschnitten Tumorzellen, die z.B. durch morphologische Veränderungen auffallen, isoliert werden. Hierzu wird der Gewebeschnitt auf die Trägerfolie gelegt. Die herauszupräparierende Zelle wird durch einen Schnitt durch das Gewebe und die Substratfolie separiert, so daß die Zelle - eventuell mit dem Substratmaterial - auf eine Unterlage transferiert wird. Diese Zellen werden anschließend histologisch analysiert. Darüber hinaus können Bakterien mit spezifischen Eigenschaften wie z.B. Zitronensäureproduzenten auf ihre Leistungsfähigkeit hin durch geeignete pH-sensitive Farbumschläge eines geeigneten Indikators erkannt und anschließend aussortiert werden.

## Patentansprüche

1. Verfahren zum Sortieren und zur Gewinnung von biologischen Objekten auf einem planaren Träger (2), auf dem sich die selektierten biologischen Objekte (10) zusammen mit weiteren biologischen Objekten befinden, **dadurch gekennzeichnet, daß** das selektierte biologische Objekt (10) von der umgebenden weiteren biologischen Masse durch einen Laserstrahl (6) abgetrennt wird, so daß das selektierte biologische Objekt (10) von seiner Umgebung freipräpariert ist, und daß anschließend das auf dem Träger (2) befindliche freipräparierte Objekt (10) durch einen weiteren Laser-Schuß von dem Träger (2) zu einer Auffangvorrichtung (18, 19) wegkatapultiert wird (Flugbahn 17).

2. Verfahren nach Anspruch 1, **dadurch gekennzeichnet,** daß ein Objektfeld (12) einer Trägerfolie (2), auf dem sich das selektierte biologische Objekt (10) befindet, mit einem Laserstrahl (6) ausgeschnitten wird und durch einen Laserschuß auf ein unmittelbar oberhalb oder unterhalb der Trägerfolie (2) angeordnetes Auffängersubstrat (5) übertragen wird.

3. Verfahren nach Anspruch 2, **dadurch gekennzeichnet,** daß der Laserstrahl (6) auf einer geschlossenen, das Objektfeld (12) einschließenden Kurve um das biologische Objekt (10) herumgeführt wird.

4. Verfahren nach Anspruch 2, **dadurch gekennzeichnet**, daß der das Objektfeld (12) einschließende Schnittbereich durch eine mit dem Laserstrahl (6) beleuchtete, auf die Trägerfolie (2) abgebildete Schlitzmaske simultan belichtet wird.

5. Verfahren nach Anspruch 2 bis 4, **dadurch gekennzeichnet**, daß das Objektfeld (12) mit dem darauf befindlichen biologischen Objekt (10) nach dem Ausschneiden über eine Distanz von 0,5 bis 10 mm, vorzugsweise 1 bis 3 mm, zu dem angeordneten Auffängersubstrat (5) transportiert wird.

6. Verfahren nach Anspruch 2 bis 5, **dadurch gekennzeichnet,** daß ein Objektfeld (12) mit einem Durchmesser von mindestens 5 µm ausgeschnitten wird.

7. Verfahren nach Anspruch 2 bis 6, **dadurch gekennzeichnet,** daß zum Ausschneiden des Objektfeldes (12) ein UV-Laser (7) verwendet wird.

8. Verfahren nach Anspruch 2 bis 7, **dadurch gekennzeichnet**, daß als Trägerfolie (2) für die biologischen Objekte (1,10) eine UV-Licht absorbierende Polymerfolie mit einer Dicke von 5 µm bis 15 µm verwendet wird.

9. Verfahren nach Anspruch 8, **dadurch gekennzeichnet,** daß das Polymer mindestens 5 Gew.-% eines aromatischen oder teilaromatischen Polykondensates enthält.

10. Verfahren nach Anspruch 1 bis 8, **dadurch gekennzeichnet,** daß als Auffängersubstrat (5) eine Folie mit einer adhäsiven Oberfläche eingesetzt wird.

11. Verfahren nach Anspruch 1 bis 10, **dadurch gekennzeichnet,** daß als Auffängersubstrat (5) eine Mikrotiterplatte mit 90 bis 500 Vertiefungen, Wells, zur Aufnahme der Proben verwendet wird.

12. Verfahren nach Anspruch 1 bis 11, **dadurch gekennzeichnet,** daß die biologischen Objekte (1, 10) mit Hilfe der Fluoreszenz-Spektroskopie erkannt und anschließend selektiert werden.

13. Verfahren nach Anspruch 1 bis 10, **dadurch gekennzeichnet,** daß biologische Objekte (1, 10) in Gewebeschnitten mit Hilfe histochemischer Farbreaktionen oder morphologischer visuell wahrnehmbarer Veränderungen erkannt und anschließend selektiert werden.

14. Verfahren nach Anspruch 2 bis 13, **dadurch gekennzeichnet,** daß die Trägerfolie (2) mit den darauf befindlichen biologischen Objekten mit einem flüssigen, für die Laserstrahlung durchlässigen Nähr- oder Puffermedium beschichtet wird.

15. Verfahren nach Anspruch 2 bis 14, **dadurch gekennzeichnet,** daß sowohl die Trägerfolie (2) mit den zu sortierenden Objekten (1, 10), als auch das Auffängersubstrat (5) in einem geschlossenen Behälter untergebracht sind, der ein UV-transparentes Fenster für den Laserstrahl besitzt.

## Claims

1. Process for the sorting and the harvesting of biological objects on a planar carrier (2) on which the selected biological objects are present together with other biological objects, **characterised in that**
the selected biological object (10) is separated from the surrounding other biological material by a laser beam (6) such that the selected biological object (10) is freed from its surroundings,
and that the freed object on the carrier (2) is subsequently catapulted away from the carrier (2) by a further laser shot to a collection device (18, 19) (trajectory 17).

2. Process according to claim 1 **characterised in that**
an object field (12) of a carrier foil (2) on which the selected biological object (10) is disposed is cut out by means of a laser beam (6) and is transferred by a laser induced transport process onto a collecting substrate (5) that is directly above or below the carrier foil (2).

3. Process according to claim 2, **characterised in that**
the laser beam is guided in a closed curve around the biological object (10) that includes the object field (12).

4. Process according to claim 2, **characterised in that**
the cut area that includes the object field (12) is simultaneously exposed through a slit mask which illuminated by the laser beam (6), and imaged on the carrier foil (2).

5. Process according to claim 2 to 4 **characterised in that**
the object field (12) with the biological object (10) disposed thereon is transported after the cutting out thereof a distance of 0.5 to 10 mm, preferable 1 to 3 mm to the provided collecting substrate (5).

6. Process according to claim 2 to 5 **characterised in that** the object field (12) is cut out with a diameter of at least 5 micro meters.

7. Process according to claim 2 to 6 **characterised in that** a UV laser (7) is used to cut out the object field (12).

8. Process according to claim 2 to 7 **characterised in that** a UV light absorbing polymer film with a thickness of 5 micro meters to 15 micro meters is used as the carrier foil (2) for the biological object (1, 10).

9. Process according to claim 8 **characterised in that** the polymer contains at least 5 weight % of an aromatic or part-aromatic polycondensate.

10. Process according to claim 1 to 8 **characterised in that** a foil with an adhesive upper surface is used as the collecting substrate (5).

11. Process according to claim 1 to 10 **characterised in that** a micro titration plate with 90 to 500 wells is used as the collecting substrate (5) for the reception of samples.

12. Process according to claim 1 to 11 **characterised in that** the biological objects (1, 10) are recognised with the help of fluorescence spectroscopy and are subsequently selected.

13. Process according to claim 1 to 10 **characterised in that** the biological objects (1, 10) are dissected tissue and are recognised with the help of histochemical colour reaction or a morphological visible change and are subsequently selected.

14. Process according to claim 2 to 13 **characterised in that** the carrier foil with the biological object thereon is coated with a fluid nutrition or buffer medium that is transparent to the laser radiation.

15. Process according to claim 2 to 14 **characterised in that** the carrier foil (2) with the objects to be sorted (1, 10) and the collection substrate (5) are housed in a closed container which has a UV transparent window for the laser beam.

## Revendications

1. Procédé de tri et d'obtention d'objets biologiques sur un support à structure plane (2) sur lequel sont situés les objets biologiques sélectionnés (10) conjointement avec d'autres objets biologiques, caractérisé en ce que l'objet biologique sélectionné (10) est séparé du reste de la masse biologique environnante au moyen d'un faisceau laser (6), de sorte que l'objet biologique sélectionné (10) est apprêté séparément de son environnement et en ce que, ensuite, l'objet (10) apprêté séparément, situé sur le support (2), est catapulté (trajectoire 17) à partir du support (2) vers un dispositif récepteur (18, 19) au moyen d'un tir supplémentaire de laser.

2. Procédé selon la revendication 1, caractérisé en ce qu'un champ d'objet (12) d'une feuille de support (2), sur lequel se trouve l'objet biologique sélectionné (10), est découpé avec un faisceau laser (6) et transféré au moyen d'un tir de laser sur un substrat récepteur (5) disposé directement au-dessus ou au-dessous de la feuille de support (2).

3. Procédé selon la revendication 2, caractérisé en ce que le faisceau laser (6) est amené autour de l'objet biologique (10) selon une courbe fermée qui englobe le champ de l'objet (12).

4. Procédé selon la revendication 2, caractérisé en ce que la zone de coupe englobant le champ de l'objet (12) est éclairée simultanément à travers un masque à fente formé sur la feuille de support (2), illuminé avec le faisceau laser (6).

5. Procédé selon l'une des revendications 2 à 4, caractérisé en ce que le champ de l'objet (12) sur lequel est situé l'objet biologique (10), après la séparation, est transporté sur une distance de 0,5 à 10 mm, de préférence de 1 à 3 mm, vers le substrat récepteur (5) associé.

6. Procédé selon l'une des revendications 2 à 5, caractérisé en ce qu'un champ d'objet (12) est découpé avec un diamètre d'au moins 5 µm.

7. Procédé selon l'une des revendications 2 à 6, caractérisé en ce qu'on utilise un laser UV (7) pour découper le champ de l'objet (12).

8. Procédé selon l'une des revendications 2 à 7, caractérisé en ce qu'on utilise, comme feuille de support (2) pour les objets biologiques (1, 10), une feuille de polymère absorbant la lumière ultraviolette, ayant une épaisseur de 5 µm à 15 µm.

9. Procédé selon la revendication 8, caractérisé en ce que le polymère contient au moins 5 % en poids d'un polycondensat aromatique ou partiellement aromatique.

10. Procédé selon l'une des revendications 1 à 8, caractérisé en ce qu'on utilise une feuille avec une surface adhésive en tant que substrat récepteur (5).

11. Procédé selon l'une des revendications 1 à 10, caractérisé en ce qu'on utilise, comme substrat récepteur (5), une plaque de microtitrage avec 90 à 500 creux pour la réception des échantillons.

12. Procédé selon l'une des revendications 1 à 11, caractérisé en ce que les objets biologiques (1, 10) sont reconnus et ensuite sélectionnés à l'aide d'une spectroscopie par fluorescence.

13. Procédé selon l'une des revendications 1 à 10, caractérisé en ce que des objets biologiques (1, 10) sont reconnus et ensuite sélectionnés dans des coupes de tissus à l'aide de réactions histochimiques avec des colorants ou à l'aide de changements visuellement perceptibles du point de vue morphologique.

14. Procédé selon l'une des revendications 2 à 13, caractérisé en ce que la feuille de support (2) sur laquelle sont situés les objets biologiques est revêtue d'un milieu liquide nutritif ou de tamponnage laissant passer le rayonnement du laser.

15. Procédé selon l'une des revendications 2 à 14, caractérisé en ce que la feuille de support (2) avec les objets à trier (1, 10) ainsi que le substrat récepteur (5) sont situés dans un conteneur fermé qui possède une fenêtre laissant passer les rayons UV pour le faisceau laser.
